(19) Europäisches Patentamt
European Patent Office
Office européen des brevets

(11) **EP 1 691 704 B1**

(12) **EUROPÄISCHE PATENTSCHRIFT**

(45) Veröffentlichungstag und Bekanntmachung des Hinweises auf die Patenterteilung:
**17.06.2015 Patentblatt 2015/25**

(51) Int Cl.:
*A61B 18/14* (2006.01)    *A61N 1/05* (2006.01)
*H01B 1/04* (2006.01)    *H01B 7/04* (2006.01)

(21) Anmeldenummer: **04803558.8**

(22) Anmeldetag: **01.12.2004**

(86) Internationale Anmeldenummer:
**PCT/EP2004/013857**

(87) Internationale Veröffentlichungsnummer:
**WO 2005/053555 (16.06.2005 Gazette 2005/24)**

(54) **ELEKTRODENLEITUNG FÜR DIE ELEKTROTHERAPIE VON HERZGEWEBE**

ELECTRODE LINE FOR THE ELECTROTHERAPY OF CARDIAC TISSUE

SONDE-ELECTRODE POUR L'ELECTROTHERAPIE DE TISSUE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **01.12.2003 DE 10356640**
**01.03.2004 DE 102004010424**

(43) Veröffentlichungstag der Anmeldung:
**23.08.2006 Patentblatt 2006/34**

(73) Patentinhaber: **Biotronik CRM Patent AG**
**6341 Baar (CH)**

(72) Erfinder:
• **HILLER, Karl-Heinz**
**97469 Gochsheim (DE)**
• **NAHRENDORF, Matthias,**
**MGH,Harvard Medical School**
**Charlestown, MA 02129 (US)**
• **BAUER, Wolfgang**
**97318 Kitzingen (DE)**

(74) Vertreter: **Lindner-Vogt, Karin L.**
**Biotronik SE & Co. KG**
**Woermannkehre 1**
**12359 Berlin (DE)**

(56) Entgegenhaltungen:
WO-A-01/97688    WO-A-02/087676
DE-A1- 19 922 999    US-A- 4 721 118
US-A- 5 336 254    US-A- 6 032 063

**Beschreibung**

Die Erfindung betrifft eine Elektrodenleitung für die Elektrotherapie oder Untersuchung von Herzgewebe, also von Myokard. An ihrem proximalen Ende weist die Elektrodenleitung einen Anschluss auf, mit dem die Elektrodenleitung mit einem elektrischen Generator, einem Elektrotherapiegerät oder auch mit einem Implantat, wie einem Herzschrittmacher oder Defibrillator verbunden ist. Das Therapiegerät ist vorzugsweise ein Generator für hochfrequenten Strom für die Gewebeablation. An ihrem distalen Ende weist die Elektrodenleitung eine oder mehrere Abfühlelektroden (Sensingelektroden) oder zusätzlich oder alternativ Behandlungselektroden auf.

Derartige Elektrodenleitungen sind im Bereich der Elektrophysiologie insbesondere zur Erfassung und Behandlung von Reizleitungsstörungen im Herzen bekannt und werden auch als EP-Katheter (Elektrophysiologie-Katheter) bezeichnet. Diese Bezeichnung soll im vorliegenden Fall sowohl Mappingkathter zum Erfassen einer myokardialen Reizleitung oder Ablationskatheter für die lokale Veröding von Herzgewebe (Myokard) oder Kombinationen solcher Elektrodenleitungen umfassen. Solche Elektrodenleitungen dienen dazu, in einem ersten Schritt die Erregungsausbreitung im Myokard mit Hilfe von Abfühlelektroden zu erfassen, um auf diese Weise Störungen der Erregungsausbreitung erkennen zu können. Anschließend kann durch Applikation von in der Regel hochfrequentem Wechselstrom, der über Behandlungselektroden gezielt abgegeben wird, eine Gewebeveröding (Ablation) herbeigeführt werden, mit der Folge, dass dort, wo das Herzgewebe verödet ist, keine Reizleitung mehr stattfindet.

Entsprechende Katheter und Therapien sind grundsätzlich bekannt und weisen bisher den großen Nachteil auf, dass die Therapie nicht unter Beobachtung im Rahmen einer Kernspintomographie (Magnetresonanztomographie) durchgeführt werden kann, da sich die elektrischen Leitungen zwischen dem proximalen Ende der Elektrodenleitung und den Elektroden am distalen Ende der Elektrodenleitung in Folge der vom Kernspintomographen induzierten Induktionsströme erheblich erwärmen können. Hinzu kommt, dass die elektrischen Leiter der üblichen Elektrodenleitungen zu unerwünschten Artefakten in dem mit dem Kernspintomographen aufgenommenen Bild führen.

Dieses Problem ist umso größer, da die maximale Leistungsaufnahme der elektrischen Leitungen dann gegeben ist, wenn deren Länge ein Vielfaches der Hälfte der vom Kernspinntomographen abgegebenen Wellenlänge, also von

$$\frac{\lambda}{2},$$

beträgt. Dies ist bei üblichen elektrischen Leitungen üblicher EP-Katheter erfüllt. Erschwerend kommt hinzu, dass die größte Erhitzung ausgerechnet in der Nähe des distalen Endes der entsprechenden Elektrodenleitung in dem Gebiet zwischen Endokard und Myokard auftritt.

Diese Gründe führen bisher dazu, dass eine elektrophysiologische Untersuchung des Myokards oder eine Elektrotherapie des Myokards in Form von Ablation oder Ähnlichem nicht unter Beobachtung im Kernspintomographen durchgeführt werden dürfen.

Ähnliche Probleme ergeben sich auch im Zusammenhang mit Stimulationselektrodenleitungen oder Defibrillationselektrodenleitungen für den Anschluss an einen implantierbaren Herzschrittmacher, Kardioverter, Defibrillator oder dergleichen. Da übliche Defibrillations- und Stimulationselektrodenleitungen nicht kernspinkompatibel sind, können die Patienten mit solchen Implantaten nicht mit einem Kernspintomographen untersucht werden.

Lediglich bekannt sind Kernspin kompatible externe Patienten kabel, die beispielsweise zur Aufnahme von EKG-Signalen genutzt werden. Diese weisen statt metallischen Leitern Kohlenfasern auf. Beispiele hierfür sind in den Dokumenten WO 01/97688 A1 und US 6,032,063 offenbart.

Ziel der Erfindung ist es daher, eine Elektrodenleitung zu schaffen, die auch eine Anwendung im Kernspintomographen erlaubt.

Erfindungsgemäß wird dieses Ziel mit einer Elektrodenleitung der eingangs genannten Art erreicht, bei dem der oder die vom proximalen zum distalen Ende der Elektrodenleitung verlaufenden elektrischen Leiter aus einem metallischen elektrischen Leiter in einem für die seitliche Auslenkung vorgesehenen Längsabschnitt mit einer Länge kürzer als ein Viertel der Wellenlänge des magnetischen Wechselfeldes eines Kernspintomographen, und einem aus Kohlenstoff-Fasern gebildeten elektrischen Leiter gebildet sind, welche miteinander verbunden sind.

Es hat sich gezeigt, dass sich mit elektrischen Leitern aus Kohlenstoff sowohl der unerwünschte Effekt der Erhitzung der elektrischen Leiter aufgrund von Wechselmagnetfeldern im Kernspintomographen induzierter Ströme als auch der unerwünschte Effekt von Artefakten in Kernspintomographen vermeiden lässt.

Interessanterweise sind aus den US-Patenten US 4,467,817, US 4,721,118, US 4,585,013 und US 5,336,254 jeweils Elektrodenleitungen zum Anschluss an einen Herzschrittmacher bekannt, bei denen der elektrische Leiter zwischen den mit dem Herzschrittmacher verbundenen proximalen Ende und dem im Herzen befindlichen distalen Ende von Kohlenstoff-Fasern gebildet sind, die in Form eines Bündels in einer Vielzahl von etwa 3000 Hochmodulfilamenten vorliegen. Mit jenen Elektrodenleitungen wurde das Ziel verfolgt, eine Elektrodenleitung möglichst geringen Durchmessers zu schaffen. In den Veröffentlichungen findet sich keinerlei Hinweis darauf, dass auch Elektrodenleitungen für die Elektrophysiologie, insbesondere Ablationselektrodenleitungen ähnlich aufgebaut sein können oder dass Elektrodenleitungen mit einem von Kohlenstoff-Fasern gebildeten elektrischen Leiter die Anwendung oder Beobachtung im Rahmen der Kernspintomographie zulassen.

Aus der WO 02/087676 A2 ist ein Ablations- und Mapping-Katheter bekannt, der im Rahmen einer MAgnetresonanztomopgrahie verwendet werden kann, indem Kohlefasern statt herkömmlichem Metall verwendet wird.Ein wesentlicher Erfindungsaspekt besteht somit in der Erkenntnis, dass Elektrodenleitungen, deren elektrische Leiter von Kohlenstoff gebildet sind, auch den Einsatz im Rahmen der Kernspintomographie erlauben.

Eine weitere, der Erfindung zugrundeliegende Erkenntnis besteht darin, dass der Einsatz von von Kohlenstoff gebildeten elektrischen Leitern gerade bei Elektrodenleitungen für den Einsatz im Rahmen der Elektrophysiologie besonders geeignet ist.

Solche Elektrodenleitungen unterscheiden sich von Stimulationselektrodenleitungen zum Anschluss an einen Herzschrittmacher zum einen dadurch, dass die Anzahl der Elektroden, die die Elektrodenleitung im Bereich ihres distalen Endes trägt, bei Elektrodenleitungen für die Elektrophysiologie typischerweise ein Mehrfaches der Anzahl von Elektroden beträgt, die eine Herzschrittmacherelektrode aufweist. Diese Vielzahl der Elektroden einer typischen Elektrodenleitung für die Elektrophysiologie ist außerdem über eine entsprechende Anzahl elektrischer Leiter mit dem proximalen Ende der Elektrodenleitung elektrisch verbunden. Eine Herzschrittmacherelektrode weist demgegenüber üblicherweise gerade einmal zwei Elektroden im Bereich des distalen Endes auf, wenn die Elektrodenleitung als bipolare Elektrodenleitung ausgebildet ist. Auch ist es für die Ablation erforderlich, über die Elektrodenleitung größere Energien zu übertragen, als dies bei Herzschrittmacherelektroden der Fall ist.

Üblicherweise sind Elektrodenleitungen für die Ablation - also Ablationskatheter steuerbar derart ausgebildet, dass ein distaler Endabschnitt der Elektrodenleitung - also des Katheterschaftes - mittels eines am proximalen Ende der Elektrodenleitung befestigten Steuergriffes seitlich auszubiegen ist. Für die dauerhafte Implantation gedachte Elektrodenleitungen zum Anschluss an einen Herzschrittmacher oder Defibrillator weisen dieses Merkmal nicht auf.

Entsprechend dem Einsatzzweck als Elektrodenleitung für die Elektrophysiologie weist eine bevorzugte Ausführungsvariante der Elektrodenleitung mehrere Ablationselektroden oder mehrere Abfühlelektroden oder beides auf. Diese Elektroden sind jeweils über einen separaten elektrischen Leiter mit einem Anschluss am proximalen Ende der Elektrodenleitung elektrisch verbunden.

Eine alternative, für den Anschluss an einen implantierbaren Defibrillator ausgebildete Elektrodenleitung weist wenigstens eine Defibrillationselektrode auf. Defibrillationselektroden unterscheiden sich von üblichen Stimulationsoder Abfühlelektroden, wie sie an einen Herzschrittmacher angeschlossen werden, durch ihre üblicherweise wesentliche größere räumliche Ausdehnung, die eine ausreichend große Elektrodenoberfläche um die Stromdichte bei den üblichen, für die Defibrillation erforderlichen Stromstärken auf ein Maß zu begrenzen, bei dem Gewebeverletzungen vermieden werden.

In allen Ausführungsvarianten ist die erfindungsgemäße Elektrodenleitung aus magnetresonanzkompatiblen Werkstoffen aufgebaut. Die Begriffe magnetresonanzkompatibel und kernspinkompatibel werden hier als Synonyme verwendet.

Die Kohlenstoff-Fasern liegen in einer bevorzugten Ausführungsvariante der Erfindung in Form von Kohlefasern vor, die eine Vielzahl von Einzelelementen umfassen.

Der elektrische Leiter ist vorzugsweise von einer isolierenden Hülle aus einem biegeweichen und magnetresonanzkompatiblem Kunststoff umhüllt. Dieser Kunststoff kann beispielsweise ganz oder teilweise aus Silikon bestehen.

Vorzugsweise enthält die isolierende Hülle einen Röntgenkontrastmittel, da weder der von Kohlenstoff gebildete elektrische Leiter noch eine isolierende Hülle beispielsweise aus Silikon einen ausreichenden Kontrast in einem Röntgenbild ergibt. Das Röntgenkontrastmittel kann beispielsweise Bariumsulfat oder Metallpartikel enthalten. Die Verteilung der Metallpartikel innerhalb einer isolierenden Kunststoffmatrix ist dabei vorzugsweise dergestalt, dass sie die Kernspinkompatibilität nicht beeinflusst und höchstens zu geringen Artefakte im Rahmen der Kernspintomographie führt.

Damit die elektrischen Leiter der Elektrodenleitung die gewünschten elektrischen Eigenschaften aufweisen, haben diese vorzugsweise eine Querschnitt zwischen 0,5 und 1,5 mm sowie eine Länge zwischen 40 cm und 120 cm. In bevorzugten Ausführungsvarianten korrespondieren dabei geringere Querschnitte mit kürzeren Längen und größere Querschnitte mit größeren Längen.

Weitere bevorzugte Ausgestaltungsmerkmale sind im Folgenden genannt.

Der metallische elektrische Leiter ist bevorzugt ein Kupferdraht.

Eine Verbindungsvariante besteht darin, dass ein Ende des metallischen Leiters überlappend zu den Kohlenstoff-Fasern des von Kohlenstoff-Fasern gebildeten elektrischen Leiters angeordnet wird und der metallische Leiter und der von Kohlenstoff-Fasern gebildete Leiter mittels einer Crimphülse kraftschlüssig verbunden werden. Ein derartiges Überlappen kann dadurch erzeugt werden, dass ein Ende des metallischen Leiters in Längsrichtung in das zunächst lockere Faserbündel geschoben wird, welches den von Kohlenstoff-Fasern gebildeten elektrischen Leiter bildet, so dass das entsprechende Ende des elektrischen Leiters vollständig von Kohlenstoff-Fasern umgeben ist.

Eine alternative Verbindungsvariante besteht darin, dass auf ein jeweiliges Ende eines von Kohlenstoff-Fasern gebildeten elektrischen Leiters eine Hülse aufgequetscht (gecrimpt) oder mit einem elektrisch leitfähigen Klebstoff aufgeklebt wird und diese Hülse durch Löten oder Schweißen mit einem weiterführenden elektrischen Leiter verbunden wird. Alternativ kann das Ende eines von Kohlenstoff-Fasern gebildeten elektrischen Leiters auch mit einem metallischen Leiter direkt verklebt sein.

Ein Erfindungsaspekt, insbesondere im Zusammenhang mit steuerbaren Ablations- oder Mappingkathetern besteht darin, dass der elektrische Leiter zwischen einer jeweiligen Elektrode und dem proximalen Ende der entsprechenden Elektrodenleitung nicht über die gesamte Länge von Kohlenstoff-Fasern gebildet ist. Vielmehr ist der elektrische Leiter in einem für die seitliche Auslenkung vorgesehenen Längsabschnitt von herkömmlichen metallischen Leitern, beispielsweise Kupferdrähten, gebildet, die dann auf die zuvor beschriebene Art und Weise mit dem von Kohlenstoff-Fasern gebildeten elektrischen Leiter verbunden sind. Da die Länge dieser metallischen elektrischen Leiter so kurz gewählt wird, dass sie kürzer ist als ein Viertel der Wellenlänge des magnetischen Wechselfeldes, kommt es trotz Einsatz eines metallischen Leiters kaum zur Erwärmung dieses metallischen Leiters, da die Länge des metallischen Leiters nicht auf die Wellenlänge des magnetischen Wechselfeldes abgestimmt ist.

Die hier angesprochenen Varianten einer Elektrodenleitung mit einem elektrischen Leiter, der längsabschnittsweise einmal von einem metallischen Leiter und zum anderen von einem von Kohlenstoff-Fasern gebildeten elektrischen Leiter gebildet ist sowie die hier angesprochenen Varianten der Verbindung eines von Kohlenstoff-Fasern gebildeten elektrischen Leiters mit einem metallischen elektrischen Leiter stellen Merkmale dar, die nicht nur bei Kathetern, wie Mappingkathetern oder Ablationskathetern, zu verwirklichen sind, sondern die auch bei Elektrodenleitungen, wie Stimulationselektrodenleitungen oder Defibrillationselektrodenleitungen zum Anschluss an implantierbare medizinische Geräte vorgesehen sein können.

Derartige Elektrodenleitungen, egal ob sie als Defibrillationselektrode zum Anschluss an einen implantierbaren Defibrillator oder als Elektrophysiologiekatheter, also als Mapping- oder Ablationskatheter ausgebildet sind, erlauben das Durchführen neuer, bisher unbekannter bzw. als undurchführbar erachteter Verfahren.

Diese Verfahren schließen Verfahren zur Elektrotherapie des Herzens ein, bei denen eine Elektrotherapie während einer Magnetresonanztomographie (Kernspintomographie) erfolgt, was durch Verwendung einer Elektrodenleitung ermöglicht wird, die unter Verwendung eines von Kohlenstoff gebildeten elektrischen Leiters magnetresonanzkompatibel aufgebaut ist.

Eine derartige Elektrotherapie umfasst vorzugsweise eine Gewebeverödung (Ablation) des Herzens durch abgabehochfrequenten Stromes an das Herzgewebe.

Alternativ oder zusätzlich kann die Elektrotherapie auch eine Elektrostimulation des Herzgewebes umfassen.

Außerdem erlauben unter Verwendung eines von Kohlenstoff gebildeten elektrischen Leiters magnetresonanzkompatibel aufgebaute Elektrodenleitungen auch ein bis dahin unbekanntes und für undurchführbar erachtetes Verfahren zum Erfassen elektrischer Potentiale von Herzgewebe, bei dem diese Potentiale während einer Magnetresonanztomographie mittels interkardial angeordneter Elektroden aufgenommen werden, wobei die Elektroden über von Kohlenstoff gebildete elektrische Leiter mit einem proximalen Ende der Elektrodenleitung elektrisch verbunden sind.

In allen hier beschriebenen Fällen können die Elektroden selbst metallisch sein oder ebenfalls von Kohlenstoff gebildet sein. Derartige Elektroden sowie der grundsätzliche Aufbau von Elektroden für die Defibrillation oder für die Elektrophysiologie sind bezüglich der Gestaltung der Elektroden und des äußeren Aufbaus der Elektrodenleitung grundsätzlich bekannt. Der entscheidende Unterschied zwischen an sich bekannten Elektrophysiologiekathetern oder Defibrillationselektrodenleitungen und der erfindungsgemäßen Elektrodenleitung besteht im Aufbau der elektrischen Leiter, die bei herkömmlichen Elektrodenleitungen, beispielsweise von Metalldrähten oder Metallwendeln, gebildet sind, während diese bei den erfindungsgemäßen Elektrodenleitungen von Kohlenstoff gebildet sind.

Im Folgenden soll ein erfindungsgemäßer Elektrophysiologiekatheter beispielhaft anhand der in den Figuren abgebildeten Ablationskathetervarianten beschrieben werden.

Figuren 1-4 zeigen einen Elektrophysiologiekatheter, der nicht gemäß der Erfindung aufgebaut ist. Von den Figuren zeigt:

Figur 1:          Eine Prinzipskizze eines steuerbaren, bipolaren Ablationskatheters:

Figur 2:          Eine Detaildarstellung des distalen Endes des Ablationskatheters aus Figur 1 in längsgeschnittener Darstellung;

Figur 3:          Einen Querschnitt durch das distale Ende des Ablationskatheters aus Figur 1, an dem in Figur 2 bezeichneten Ort;

Figur 4:          Einen Längsschnitt eines gegenüber Figur 3 alternativen Aufbaus des distalen Endes eines Ablationskatheters, wie aus Figur 1;

Figuren 5a) bis d):     Vier aufeinanderfolgende Längsabschnitte eines erfindungsgemäßen Katheteraufbaus in längsgeschnittener Darstellung; und

Figuren 5e) bis g):       Darstellungen der in Figur 5b) bezeichneten Querschnitte.

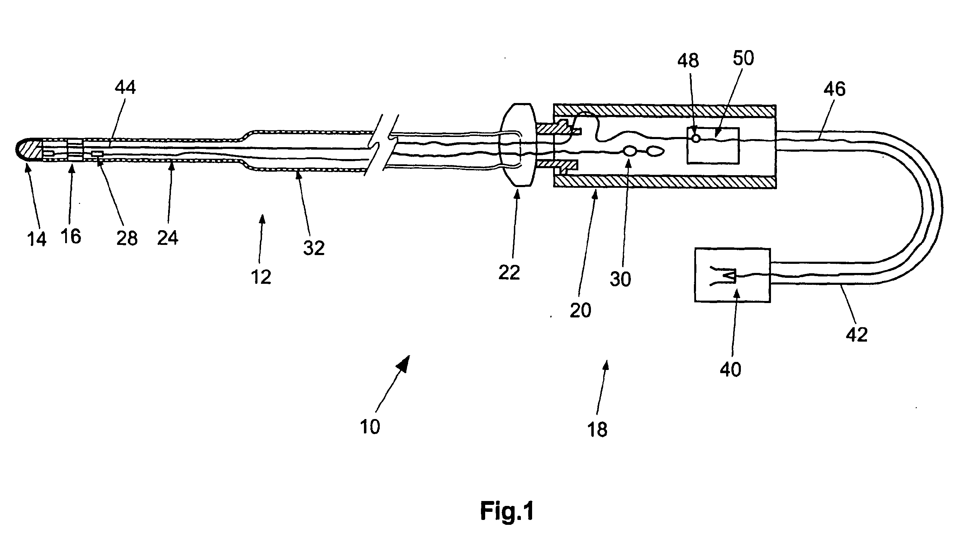

Der in Figur 1 dargestellte Ablationskatheter 10 besitzt einen Katheterschaft 12, der im Rahmen dieser Beschreibung und der Ansprüche auch als Elektrodenleitung 12 bezeichnet wird. Die Elektrodenleitung 12 trägt an ihrem distalen Ende zwei Elektroden, nämlich eine Tipp-Elektrode 14 und eine Ringelektrode 16. Die Tipp-Elektrode 14 und die Ringelektrode 16 dienen im Falle der Gewebeveröldung (Ablation) der Abgabe hochfrequenten Wechselstroms an umliegendes Gewebe, um das Gewebe mittels des Wechselstroms so weit zu erhitzen, dass es verödet.

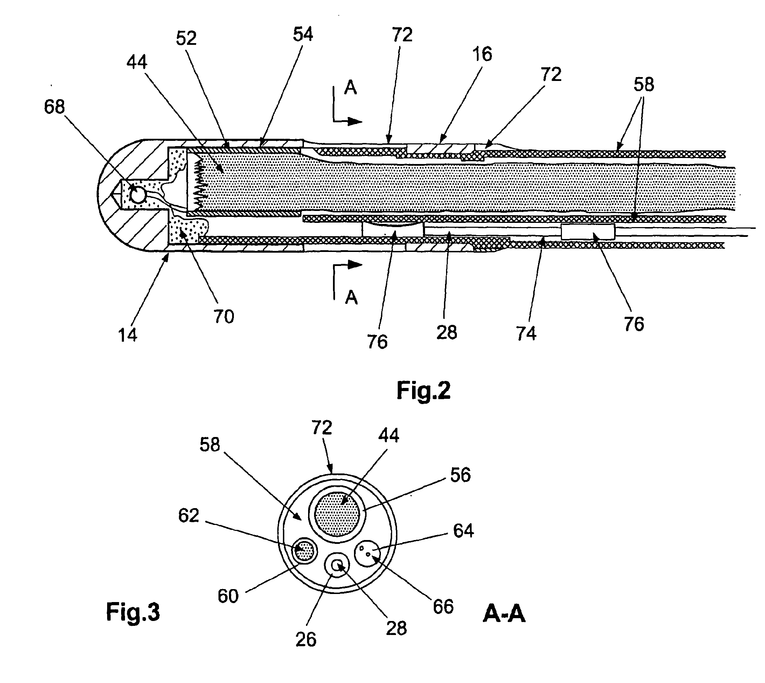

Am proximalen Ende ist die Elektrodenleitung 12 mit einem Handgriff 18 verbunden, der ein Haltestück 20 und ein Schiebestück 22 aufweist. Haltestück 20 und Schiebestück 22 sind in bekannter Weise in Längsrichtung des Haltestücks 20 relativ zueinander verschiebbar, um eine laterale Ausbiegung eines distalen Endabschnitts 24 der Elektrodenleitung 12 zu bewirken. Dies geschieht in grundsätzlich bekannter Weise mit Hilfe eines in einem Lumen 26 (siehe Figur 3) der Elektrodenleitung 12 geführten Steuerdrahtes 28, der im Bereich des distalen Endes der Elektrodenleitung 12 befestigt ist. Proximal dieser Befestigungsstelle ist der Steuerdraht 28 längsverschieblich in dem Lumen 26 geführt und über eine Crimpverbindung 30 fest mit dem Haltestück 20 verbunden, während das proximale Ende der Elektrodenleitung 12 im übrigen mit dem Schiebestück 22 verbunden ist. Auf diese dem Fachmann bekannte Weise lässt sich mit Hilfe des Haltestückes 20 des Schiebestückes 22 eine relative Verschiebung des Steuerdrahtes 28 gegenüber der übrigen Elektrodenleitung 12 bewirken, die wegen der in Bezug auf den Querschnitt der Elektrodenleitung 12 exzentrischen Anordnung des Steuerdrahtes 28 zu einer entsprechenden lateralen Ausbiegung des distalen Endabschnitts 24 der Elektrodenleitung 12 führt. Diese Form der Steuerbarkeit ist ein dem Fachmann bekanntes Merkmal üblicher Ablationskatheter.

Damit sich eine laterale Ausbiegung des distalen Endabschnitts 24 der Elektrodenleitung 12 auf diesen distalen Endabschnitt 24 beschränkt, ist der distale Endabschnitt 24 biegeweicher aufgebaut, als ein daran anschließender proximaler Schaftabschnitt 32 der Elektrodenleitung 12. Der proximale Schaftabschnitt ist durch ein Geflecht verstärkt.

Wie erwähnt, dienen die Elektroden 14 und 16 der Abgabe hochfrequenten Wechselstroms für die Ablation oder auch dem Abfühlen elektrischer Potentiale des Herzgewebes, also dem Mapping. Zu beiden Zwecken müssen die Elektroden 14 und 16 elektrisch mit einem Anschluss 40 verbunden sein, der über ein Kabel 42 mit dem Handgriff 18 verbunden ist.

Die elektrische Verbindung zwischen der jeweiligen Elektrode 14 bzw. 16 und dem Anschluss 40 ist im Folgenden beispielhaft nur für die elektrische Verbindung zwischen der Tipp-Elektrode 14 und dem Anschluss 40 beschrieben. Die elektrische Verbindung zwischen der Ringelektrode 16 und dem Anschluss 40 ist entsprechend gestaltet.

Die Tipp-Elektrode 14 ist über einen von Kohlenstoff-Fasern (Carbon-Fasern) gebildeten elektrischen Leiter 44 mit einem üblichen elektrischen Leiter 46 des Kabels 42 verbunden. Der von Kohlenstoff-Fasern gebildete elektrische Leiter 44 erstreckt sich von der Tipp-Elektrode 14 bis zu einer Verbindungsstelle innerhalb des Haltestückes 20, die von einer auf das proximale Ende des von Kohlenstoff-Fasern gebildeten elektrischen Leiters 44 aufgeschoben, wenn Crimphülse 48 und einer kleinen Platine 50 gebildet ist.

Die Crimphülse 48 ist in bekannter Weise durch Quetschen mit dem proximalen Ende des von Kohlenstoff-Fasern gebildeten elektrischen Leiters 44 verbunden. Darüber hinaus ist die Crimphülse 48 auf eine Leiterbahn in der Platine 50 aufgelötet oder mit dieser Leiterbahn verschweißt. In gleicher Weise ist der übliche elektrische Leiter 46 mit derselben Leiterbahn der Platine 50 verbunden.

Die elektrische Verbindung zwischen der Tipp-Elektrode 14 und dem distalen Ende des von Kohlenstoff-Fasern gebildeten elektrischen Leiters 44 ist wie folgt aufgebaut: Auf das distale Ende des elektrischen Leiters 44 ist eine distale Crimphülse 52 aufgequetscht. Diese metallische distale Crimphülse 52 ist über eine Löt- oder Schweißverbindung 54 elektrisch mit der kappförmig gestalteten Tipp-Elektrode 14 elektrisch verbunden.

Wie dem in Figur 3 abgebildeten Querschnitt A-A zu entnehmen ist, ist der von Kohlenstoff-Fasern gebildete elektrische Leiter 44 in einem weiteren Lumen 56 eines Multilumenschlauches 58 geführt. Dieser Multilumenschlauch 58 erstreckt sich zwischen dem distalen und dem proximalen Ende der Elektrodenleitung 12. Der Multilumenschlauch 58 ist aus biegeweichen, elektrisch isolierendem Kunststoff gefertigt. Ein geeigneter Kunststoff ist beispielsweise Silicon. Neben den bereits erwähnten Lumina 26 und 56 für den Steuerdraht 28 bzw. den von Kohlenstoff-Fasern gebildeten elektrischen Leiter 44 weist der Multilumenschlauch 58 zwei weitere Lumina auf, nämlich ein Lumen 60, in dem ein weiterer, von Kohlenstoff-Fasern gebildeter elektrischer Leiter 62 geführt ist, der der Kontaktierung der Ringelektrode 16 dient und einen geringeren Durchmesser aufweist, als der elektrische Leiter 44 zur Kontaktierung der Tipp-Elektrode 14.

Ein viertes Lumen 64 dient der Aufnahme von zwei elektrischen Leitern oder zwei lichtleitenden Fasern 66, die zu einem Temperatursensor 68 führen, welcher in einem Hohlraum im distalen Ende der Tipp-Elektrode 14 angeordnet ist. Mit Hilfe des Temperatursensors 68 kann eine Gewebeablation in an sich bekannter Weise temperaturgesteuert durchgeführt werden. Der Temperatursensor 68 ist mittels eines elektrisch isolierenden, aber wärmeleitenden Klebstoffs 70 mit der Tipp-Elektrode 14 verbunden. Aus Gründen der Magnetresonanzkompatibilität ist der Temperatursensor 68 vorzugsweise ein optischer Temperatursensor, der über zwei lichtleitende Fasern mit dem proximalen Ende der Elektrodenleitungen 12 verbunden ist. Falls alternativ ein elektrischer Temperatursensor, wie beispielsweise ein Thermoelement verwendet werden sollte, müsste dieser Sensor mit zwei elektrischen Leitern mit dem proximalen Ende der Elektrodenleitung 12 verbunden sein. Diese elektrischen Leiter sind vorzugsweise metallische Leiter. Sie können jedoch so dünn ausgeführt sein und thermisch so gut gegenüber dem Äußeren der Elektrodenleitung 12 isoliert sein, dass eine Erwärmung dieser Leiter 66 im Rahmen der Magnetresonanztomographie unbeachtlich ist.

Bei der dargestellten Elektrodenkonfiguration dient die Tipp-Elektrode 14 sowohl als Abfühlelektrode als auch als (unipolare) Ablationselektrode, während die Ringelektrode 16 ausschließlich als Abfühlelektrode dient. Daher kann der elektrische Leiter 62 auch ein geringeres Querschnittsmaß aufweisen als der elektrische Leiter 44.

Die Ringelektrode 16 ist außen auf den Multilumenschlauch 58 aufgeschoben. In gleicher Weise ist die kappenförmige Tipp-Elektrode 14 auf das distale Ende des Multilumenschlauches 58 aufgeschoben. Für die Kontaktierung der jeweiligen Elektroden 14 bzw. 16 weisen die entsprechenden Lumina 56 bzw. 60 im Bereich der Elektroden 14 und 16 laterale Öffnungen auf (in den Figuren nicht dargestellt). Eine umlaufende Vertiefung, die sich außen auf die Elektrodenleitung 12 in einem zwischen den beiden Elektroden 14 und 16 befindlichen Längsabschnitt ergibt, ist mit einem UV-härtenden Kunststoff 72 aufgefüllt. In gleicher Weise ist der Übergang vom proximalen Ende der Ringelektrode 16 zur Außenoberfläche des Multilumenschlauches 58 mit UV-härtendem Kunststoff 72 verfüllt.

Wie zuvor beschrieben, ist der Steuerdraht 28 an seinem distalen Ende mit der Elektrodenleitung 12 verbunden, damit der Steuerdraht 28 als exzentrisch angeordneter Zugdraht eine seitliche Ausbiegung des distalen Endabschnitts 24 der Elektrodenleitung 12 bewirken kann. Die dazu erforderliche Verbindung des distalen Endes des Steuer- oder Zugdrahts 28 mit der Elektrodenleitung 12 ist durch eine Verklebung des distalen Endes des Steuerdrahtes 28 mit dem Lumen 26 im Bereich des distalen Endes des Steuerdrahtes 28 gelöst. Dazu ist der Zwischenraum zwischen dem Steuerdraht 28 und der Wand des Lumens 26 mit einem Klebstoff 74 ausgefüllt. Damit dieser Klebstoff 74 auch im ungehärteten Zustand am Ort der Verklebung bleibt, sind zwei Schlauchabschnitte 76 so in das Lumen 26 ein- und auf den Steuerdraht 28 aufgeschoben, dass die Schlauchabschnitte 26 einen Längsabschnitt voneinander haben und zwischen sich die beabsichtigte Klebestelle dichtend einschließen.

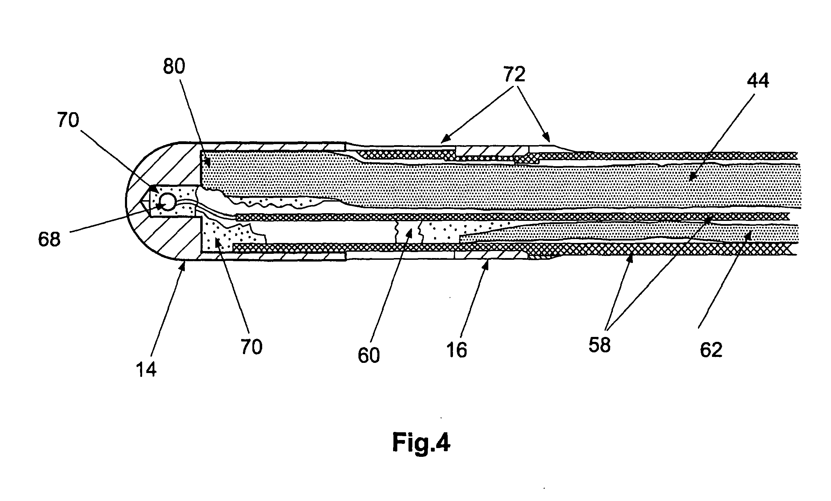

Wie erwährt, stellt Figur 4 einen zu Figur 2 alternativen Aufbau des distalen Endes der Elektrodenleitung 12 in einem vergrößert dargestelltem Längsschnitt dar. Die in Figur 4 dargestellte Variante unterscheidet sich von der in Figur 2 dargestellten dadurch, dass die Verbindung der von Kohlenstoff-Fasern gebildeten elektrischen Leiter 44 und 62 mit den entsprechenden Elektroden 14 bzw. 16 nicht mit Hilfe von Crimphülsen erfolgt, sondern mittels eines elektrisch leitenden Klebstoffs, mit dem die distalen Enden der elektrischen Leiter 62 und 44 mit der Ringelektrode 16 bzw. der Tipp-Elektrode 14 verklebt sind. Der übrige Aufbau entspricht den in Figur 2 dargestellten.

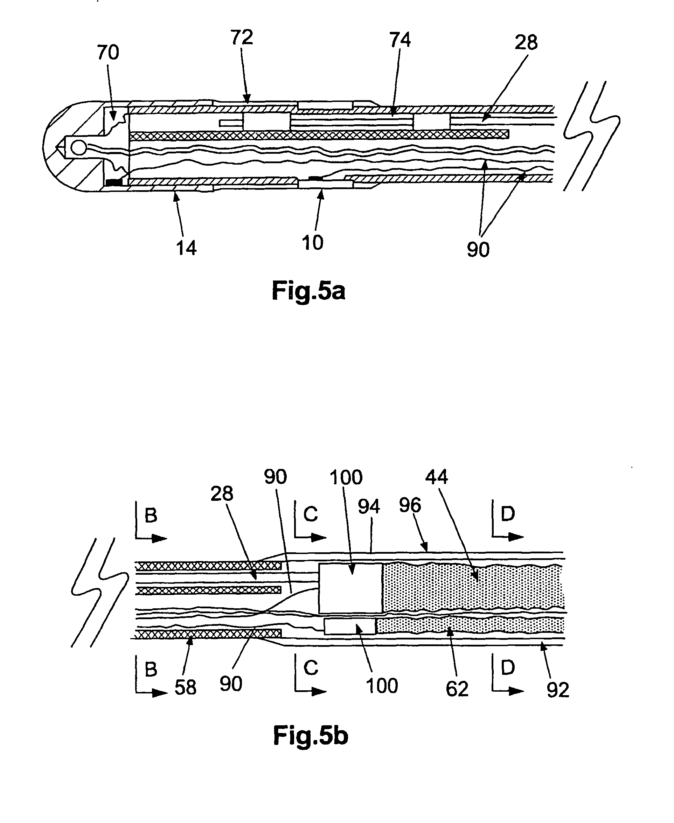

In den Figuren 5a) bis g) sind mehrere Längs- und Querschnitte einer erfindungsgemäßen Elektrodenleitung dargestellt, die sich von den zuvor dargestellten Elektroden dadurch unterscheidet, dass die von Kohlenstoff-Fasern gebildeten elektrischen Leiter 44 und 62 nur innerhalb des relativ biegesteifen, proximalen Längsabschnitts der Elektrodenleitung verlaufen, während die elektrischen Leiter im biegeweichen distalen Längsabschnitt der Elektrodenleitung herkömmliche Kupferleiter 90 sind, siehe Figur 5a) und 5b).

In Figur 5b) ist derjenige Längsabschnitt der Elektrodenleitung im Längsschnitt dargestellt, in dem die Elektrodenleitung von einem proximalen, relativ biegesteifen proximalen Schaftabschnitt zu dem biegeweichen, distalen Schaftabschnitt übergeht. Die Steifigkeit des proximalen Schaftabschnitts wird unter anderem durch ein schlauchartiges Drahtgeflecht 92 bewirkt, welches am distalen Ende mit einer Metallhülse 94 im Bereich einer Schweißstelle 96 verschweißt ist. In die Metallhülse 94 ist der Multilumenschlauch 58 eingeklebt.

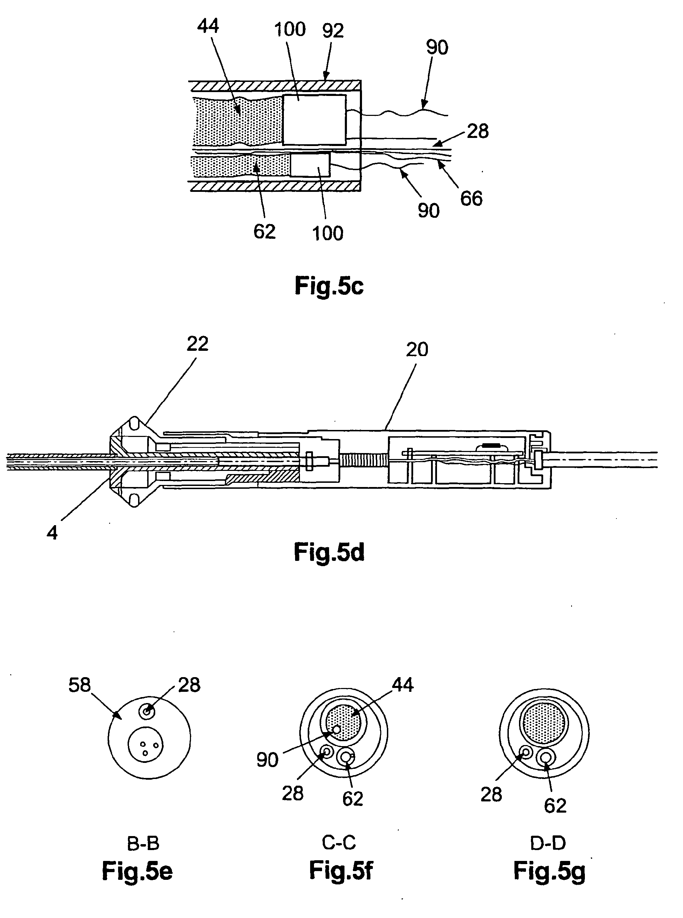

Sowohl an ihrem jeweiligen proximalen Ende als auch an ihrem jeweiligen distalen Ende sind die von Kohlenstoff-Fasern gebildeten Leiter 44 und 62 mit herkömmlichen Kupferleitern 90 verbunden. Diese Verbindung ist bei der Ausführungsvariante gemäß Figur 5 jeweils dergestalt, dass ein jeweiliges Ende der Kupferleiter 90 dabei zwischen die den jeweiligen elektrischen Leiter 44 bzw. 62 bildenden Kohlenstoff-Fasern ragt, wie in dem Querschnitt gemäß Figur 5f) dargestellt ist. Ein Herausrutschen des Kupferleiterendes wird jeweils durch eine Crimphülse 100 verhindert, mit der die Kohlenstoff-Fasern der elektrischen Leiter 44 und 62 und die jeweiligen Enden der Kupferleiter 90 miteinander verquetscht und somit kraftschlüssig miteinander verbunden sind.

Der Vorteil der in Figur 5 abgebildeten Ablationskathetervariante gegenüber den zuvor beschriebenen Ablationskathetervarianten besteht darin, dass der flexible, distale Endabschnitt der Elektrodenleitung durch den Einsatz metallischer Leiter, konkret durch den Einsatz von Kupferkabeln, eine größere Flexibilität aufweist und sich daher besser seitlich ausbiegen und genauer steuern lässt. Trotz des Einsatzes metallischer Leiter bei der Ausführungsvariante gemäß Figur 5 ist auch der in Figur 5 abgebildete Ablationskatheter magnetresonanztauglich, da die elektrischen Leiter eine Länge besitzen, die kürzer ist als ein Viertel der Wellenlänge eines typischen magnetischen Wechselfeldes, wie es in der Magnetresonanztomographie eingesetzt wird. Die ansonsten durch eine Art Antennenwirkung bedingte Erwärmung von metallischen elektrischen Leitern in solchen Wechselfeldern findet dann aufgrund der Fehlanpassung nur in sehr geringem, unbeachtlichen Maße statt.

Der übrige Aufbau der Elektrodenleitung gemäß Figur 5 ergibt sich analog zu den zuvor beschriebenen Beispielen. Dementsprechend sind sich entsprechende Bauteile mit denselben Bezugzeichen versehen, wie in den vorangegangen Figuren.

**Patentansprüche**

1.  Elektrodenleitung für die Defibrillation oder für das Mapping oder die Ablation von Herzgewebe, mit einem Anschluss an einem proximalen Ende der Elektrodenleitung und einer oder mehreren Abfühl- oder Behandlungselektroden oder beidem an oder nahe einem distalen Ende der Elektrodenleitung, sowie mit wenigstens einem elektrischen Leiter, über den eine jeweilige Abfühl- oder Behandlungselektrode mit dem Anschluss elektrisch verbunden ist, wobei die Elektrodenleitung für die Verwendung im Rahmen einer Magnetresonanztomographie tauglich aufgebaut sowie zum Anschluss an ein Elektrophysiologie-Therapiegerät ausgebildet ist und wenigstens eine Defibrillationselektrode oder wenigstens eine Abfühlelektrode für die Aufnahme und Auswertung von Herzgewebepotentialen oder wenigstens eine Behandlungselektrode für die Abgabe von Hochfrequenzströmen zur Gewebeverödung (Ablation) aufweist,
    **dadurch gekennzeichnet,**
    **dass** der elektrische Leiter aus mindestens einem metallischen elektrischen Leiter in einem für die seitliche Auslenkung vorgesehenen Längsabschnitt mit einer Länge kürzer als ein Viertel der Wellenlänge des magnetischen Wechselfeldes eines Kernspintomographen, und mindestens einem aus Kohlenstoff-Fasern gebildeten elektrischen Leiter gebildet ist, welche miteinander verbunden sind.

2.  Elektrodenleitung nach Anspruch 1, **dadurch gekennzeichnet, dass** der mindestens eine aus Kohlenstoff-Fasern gebildete elektrische Leiter von Kohlefasern gebildet ist, die eine Vielzahl von Filamenten umfassen.

3.  Elektrodenleitung nach Anspruch 1, **dadurch gekennzeichnet, dass** der mindestens eine metallische elektrische Leiter ein Kupferdraht ist.

4.  Elektrodenleitung nach einem der Ansprüche 1 bis 3, **dadurch gekennzeichnet, dass** der elektrische Leiter von einer isolierenden Hülle aus einem biegeweichen und magnetresonanzkompatiblen Kunststoff umhüllt ist

5.  Elektrodenleitung nach Anspruch 4, **dadurch gekennzeichnet, dass** die isolierende Hülle ein Röntgenkontrastmittel enthält.

6.  Elektrodenleitung nach Anspruch 5, **dadurch gekennzeichnet, dass** das Röntgenkontrastmittel Bariumsulfat oder Metallpartikel enthält.

7.  Elektrodenleitung nach einem der Ansprüche 4 bis 6, **dadurch gekennzeichnet, dass** die isolierende Hülle zum größten Teil von Silikon gebildet ist.

8.  Elektrodenleitung nach einem der Ansprüche 1 bis 3, **dadurch gekennzeichnet, dass** der elektrische Leiter einen Querschnitt zwischen 0,5 mm und 1,5 mm sowie eine Länge zwischen 40 und 120 cm besitzt.

9.  Elektrodenleitung nach Anspruch 2, **dadurch gekennzeichnet, dass** die Filamente der Kohlenstoff-Fasern einen Durchmesser zwischen 5 $\mu$m und 7 $\mu$m besitzen.

10. Elektrodenleitung nach Anspruch 1, **dadurch gekennzeichnet, dass** ein Ende des mindestens einen metallischen elektrischen Leiters überlappend zu den Kohlenstoff-Fasern des mindestens einen aus Kohlenstoff-Fasern gebildeten elektrischen Leiters angeordnet ist und der mindestens eine metallische elektrische Leiter und der mindestens eine aus Kohlenstoff-Fasern gebildete elektrische Leiter mittels einer Crimphülse kraftschlüssig verbunden sind.

11. Elektrodenleitung nach Anspruch 10, **dadurch gekennzeichnet, dass** ein Ende des mindestens einen metallischen Leiters in Längsrichtung in das Faserbündel geschoben ist, welches den aus Kohlenstoff-Fasern gebildeten elektrischen Leiter bildet, so dass das entsprechende Ende des mindestens einen elektrischen Leiters vollständig von Kohlenstoff-Fasern umgeben ist und eine Überlappung erzeugt ist.

12. Elektrodenleitung nach Anspruch 1, **dadurch gekennzeichnet, dass** auf ein Ende des aus Kohlenstoff-Fasern gebildeten elektrischen Leiters eine Hülse aufgequetscht (gecrimpt) oder mit einem elektrisch leitfähigen Klebstoff aufgeklebt ist und diese Hülse durch Löten oder Schweißen mit dem weiterführenden metallischen elektrischen Leiter verbunden ist.

13. Elektrodenleitung nach Anspruch 1, **dadurch gekennzeichnet, dass** das Ende des mindestens einen aus Kohlenstoff-Fasern gebildeten elektrischen Leiters direkt mit dem mindestens einen metallischen elektrischen Leiter mittels eines elektrisch leitfähigen Klebstoffes verklebt ist.

14. Elektrodenleitung nach Anspruch 1, **dadurch gekennzeichnet, dass** die Elektrodenleitung als Defibrillationselektrode zum Anschluss an einen implantierbaren Defibrillator ausgebildet ist.

15. Elektrodenleitung nach Anspruch 1, **dadurch gekennzeichnet, dass** die Elektrodenleitung als Elektrophysiologiekatheter für das Mapping oder die Ablation von Herzgewebe oder beidem ausgebildet ist.

**Claims**

1. An electrode line for the defibrillation or for the mapping or the ablation of heart tissue, comprising a connector at a proximal end of the electrode line and one or more sensing or treatment electrodes or both at or in the vicinity of a distal end of the electrode line, and also comprising at least one electrical conductor, via which a respective sensing or treatment electrode is electrically connected to the connector, wherein the electrode line is structured to be suitable for use within the scope of magnetic resonance imaging and is also configured for attachment to an electrophysiology therapy device and has at least one defibrillation electrode or at least one sensing electrode for recording and evaluating heart tissue potentials or at least one treatment electrode for delivering high-frequency currents for tissue sclerotherapy (ablation),
**characterised in that**
the electrical conductor is formed from at least one metal electrical conductor in a longitudinal portion intended for lateral deflection and having a length shorter than a quarter of the wavelength of the magnetic alternating field of a magnetic resonance scanner, and from at least one electrical conductor formed from carbon fibres, said electrical conductors being connected to one another.

2. The electrode line according to Claim 1, **characterised in that** the at least one electrical conductor formed from carbon fibres is formed by carbon fibres that comprise a plurality of filaments.

3. The electrode line according to Claim 1, **characterised in that** the at least one metal electrical conductor is a copper wire.

4. The electrode line according to one of Claims 1 to 3, **characterised in that** the electrical conductor is encased by an insulating sleeve made of a flexible plastic compatible with magnetic resonance.

5. The electrode line according to Claim 4, **characterised in that** the insulating sleeve contains an X-ray contrast agent.

6. The electrode line according to Claim 5, **characterised in that** the X-ray contrast agent contains barium sulphate or metal particles.

7. The electrode line according to one of Claims 4 to 6, **characterised in that** the insulating sleeve is formed for the majority by silicone.

8. The electrode line according to one of Claims 1 to 3, **characterised in that** the electrical conductor has a cross section between 0.5 mm and 1.5 mm and also a length between 40 and 120 cm.

9. The electrode line according to Claim 2, **characterised in that** the filaments of the carbon fibres have a diameter between 5 $\mu$m and 7 $\mu$m.

10. The electrode line according to Claim 1, **characterised in that** one end of the at least one metal electrical conductor is arranged in a manner overlapping the carbon fibres of the at least one electrical conductor formed from carbon fibres, and the at least one metal electrical conductor and the at least one electrical conductor formed from carbon fibres are connected in a force-fitting manner by means of a crimp sleeve.

11. The electrode line according to Claim 10, **characterised in that** one end of the at least one metal conductor is slid in the longitudinal direction into the fibre bundle, which forms the electrical conductor formed from carbon fibres, such that the corresponding end of the at least one electrical conductor is completely surrounded by carbon fibres and an overlap is produced.

**12.** The electrode line according to Claim 1, **characterised in that** a sleeve is squeezed (crimped) onto an end of the electrical conductor formed from carbon fibres or is adhesively bonded thereto using an electrically conductive adhesive, and this sleeve is connected by soldering or welding to the continuing metal electrical conductor.

**13.** The electrode line according to Claim 1, **characterised in that** the end of the at least one electrical conductor formed from carbon fibres is directly bonded to the at least one metal electrical conductor by means of an electrically conductive adhesive.

**14.** The electrode line according to Claim 1, **characterised in that** the electrode line is configured as a defibrillation electrode for attachment to an implantable defibrillator.

**15.** The electrode line according to Claim 1, **characterised in that** the electrode line is configured as an electrophysiology catheter for the mapping or the ablation of heart tissue or both.

**Revendications**

**1.** Fil d'électrode pour la défibrillation ou pour le mappage ou l'ablation de tissus cardiaques, avec une borne à une extrémité proximale du fil d'électrode et une ou plusieurs électrodes de détection ou de traitement ou les deux à une extrémité distale du fil d'électrode ou à proximité de celle-ci, et avec au moins un conducteur électrique par lequel une électrode de détection ou de traitement respective est électriquement reliée à la borne, le fil d'électrode étant adapté à une utilisation dans le cadre d'une tomographie par résonance magnétique et conçu pour être raccordé à un dispositif thérapeutique d'électrophysiologie, et comporte au moins une électrode de défibrillation ou au moins une électrode de détection pour l'enregistrement et l'évaluation de potentiels des tissus cardiaques ou au moins une électrode de traitement pour la délivrance de courants à haute fréquence en vue d'une sclérose des tissus (ablation),
**caractérisé en ce que**
le conducteur électrique est constitué d'au moins un conducteur électrique métallique, dans un segment longitudinal prévu pour la déviation latérale, d'une longueur inférieure à un quart de la longueur d'onde du champ magnétique alternatif d'un tomographe à spin nucléaire, et d'au moins un conducteur électrique en fibres de carbone, lesquels sont reliés l'un à l'autre.

**2.** Fil d'électrode selon la revendication 1, **caractérisé en ce que** l'au moins un conducteur électrique en fibres de carbone est constitué de fibres de carbone comprenant une multitude de filaments.

**3.** Fil d'électrode selon la revendication 1, **caractérisé en ce que** l'au moins un conducteur électrique métallique est un fil de cuivre.

**4.** Fil d'électrode selon l'une des revendications 1 à 3, **caractérisé en ce que** le conducteur électrique est enrobé dans une gaine isolante constituée d'une matière plastique flexible et compatible avec la résonnance magnétique.

**5.** Fil d'électrode selon la revendication 4, **caractérisé en ce que** la gaine isolante contient un agent de contraste radiologique.

**6.** Fil d'électrode selon la revendication 5, **caractérisé en ce que** l'agent de contraste radiologique contient du sulfate de baryum ou des particules métalliques.

**7.** Fil d'électrode selon l'une des revendications 4 à 6, **caractérisé en ce que** la gaine isolante est constituée majoritairement de silicone.

**8.** Fil d'électrode selon l'une des revendications 1 à 3, **caractérisé en ce que** le conducteur électrique présente une section transversale comprise entre 0,5 mm et 1,5 mm et une longueur comprise entre 40 et 120 cm.

**9.** Fil d'électrode selon la revendication 2, **caractérisé en ce que** les filaments des fibres de carbone présentent un diamètre compris entre 5 $\mu$m et 7 $\mu$m.

**10.** Fil d'électrode selon la revendication 1, **caractérisé en ce qu'**une extrémité de l'au moins un conducteur électrique métallique est agencé de manière à chevaucher les fibres de carbone de l'au moins un conducteur électrique composé de fibres de carbone, et **en ce que** l'au moins un conducteur électrique métallique et l'au moins un conducteur électrique composé de fibres de carbone sont reliés par combinaison de forces au moyen d'un manchon de sertissage.

**11.** Fil d'électrode selon la revendication 10, **caractérisé en ce qu'**une extrémité de l'au moins un conducteur métallique est enfoncée dans la direction longitudinale dans le faisceau de fibres formant le conducteur électrique composé de fibres de carbone, de manière à ce que l'extrémité correspondante de l'au moins un conducteur électrique soit entourée complètement de fibres de carbone et de manière à créer un chevauchement.

**12.** Fil d'électrode selon la revendication 1, **caractérisé en ce qu'**un manchon est fixé (serti) sur une extrémité de conducteur électrique composé de fibres de carbone, ou collé à l'aide d'une colle électriquement conductrice, et **en ce que** ce manchon est relié au conducteur électrique métallique de transmission par soudure ou brasure.

**13.** Fil d'électrode selon la revendication 1, **caractérisé en ce que** l'extrémité de l'au moins un conducteur électrique composé de fibres de carbone est directement collée sur l'au moins un conducteur électrique métallique au moyen d'une colle électriquement conductrice.

**14.** Fil d'électrode selon la revendication 1, **caractérisé en ce que** le fil d'électrode est conçue comme une électrode de défibrillation destinée à être raccordée à un défibrillateur implantable.

**15.** Fil d'électrode selon la revendication 1, **caractérisé en ce que** le fil d'électrode est conçu comme un cathéter d'électrophysiologie pour le mappage ou l'ablation de tissus cardiaques ou les deux.

**Fig.1**

Fig.2

Fig.3

A-A

Fig.4

**Fig.5a**

**Fig.5b**

**Fig.5c**

**Fig.5d**

B-B
**Fig.5e**

C-C
**Fig.5f**

D-D
**Fig.5g**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0197688 A1
- US 6032063 A
- US 4467817 A
- US 4721118 A
- US 4585013 A
- US 5336254 A
- WO 02087676 A2